# EUROPEAN PATENT APPLICATION

(11) **EP 4 837 903 A1**
(43) Date of publication of application: **11.11.2026**
(21) Application number: 25751729.2
(22) Date of filing: 08.02.2025
(51) Int. Cl.: C07D 401/10, A61K 31/454, A61P 11/00, A61P 1/08

(54) **TETRAZOLE COMPOUND, PHARMACEUTICAL COMPOSITION THEREOF AND USE THEREOF**

(30) Priority: 08.02.2024 CN 202410177070
(71) Applicant: SHANGHAI SHUYIN XINKE INTELLIGENT TECHNOLOGY CO., LTD., Shanghai 201109 (CN)
(72) Inventor: YUAN, Ye, Shanghai 201109 (CN)
(74) Representative: Chung, Hoi Kan
(86) International application number: PCT/CN2025/076521
(87) International publication number: WO 2025/168119

(57) **Abstract**

Disclosed in the present invention are a tetrazole compound, a pharmaceutical composition thereof, and the use thereof. Specifically disclosed in the present invention are compound I, a pharmaceutically acceptable salt thereof, a solvate thereof, or a solvate of a salt thereof, which have one or more of the following advantages: novel structure, good metabolic stability, high bioavailability, and effectively alleviating pathological damage caused by pulmonary fibrosis.

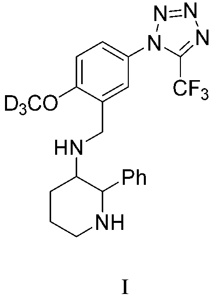

## Description

### TECHNICAL FIELD

The present invention relates to a tetrazole compound, a pharmaceutical composition thereof, and uses thereof.

### BACKGROUND ART

Pulmonary fibrosis (PF) is a disease characterized by diffuse pneumonia and alveolar structural disorder, ultimately leading to interstitial pulmonary fibrosis. It is currently generally believed that the pathogenesis of pulmonary fibrosis is mainly characterized by persistent alveolar epithelial cell injury, abnormal activation of fibroblasts, and excessive deposition of large amounts of extracellular matrix, thereby leading to varying degrees of inflammation and fibrosis in the alveoli and pulmonary interstitium, and consequently resulting in structural lung damage and functional loss.

Idiopathic pulmonary fibrosis (IPF) is the most common and severe chronic interstitial lung disease of unknown etiology. Clinically, it manifests as progressive dyspnea accompanied by irritating dry cough. The condition often continues to progress, with a median survival time of approximately 3-5 years.

According to statistics, 5 million people worldwide suffer from IPF, and this number is continuously increasing due to the aggravation of pulmonary injury factors such as environmental pollution. Currently, treatment options for IPF are very limited, and there are no drugs that can significantly prolong patient survival. During this period, patients' quality of life and psychological state are severely affected due to the emergence of various complications.

Currently, the two orally administered anti-pulmonary fibrosis drugs recommended for clinical use and approved by the U.S. Food and Drug Administration (FDA) are pirfenidone and nintedanib. The anti-pulmonary fibrosis mechanism of Pirfenidone (PFD) mainly involves regulating pro-fibrotic cytokines such as transforming growth factor-β (TGF-β) and platelet-derived growth factor (PDGF), reducing the biological activity of fibroblasts, inhibiting fibroblast proliferation, and decreasing the expression of collagen fibers and the synthesis and accumulation of extracellular matrix. Nintedanib is a small-molecule tyrosine kinase inhibitor that can simultaneously block the signal transduction pathways of vascular endothelial growth factor receptor (VEGFR), platelet-derived growth factor receptor (PDGFR), and fibroblast growth factor receptor (FGFR), exhibiting anti-fibrotic and anti-inflammatory effects.

Although these two marketed drugs can slow down the rate of decline in lung function, they cannot reverse disease progression. Moreover, pirfenidone treatment is associated with side effects such as photosensitivity reactions, anorexia, dizziness, and abdominal discomfort; common adverse reactions of nintedanib include diarrhea, nausea, vomiting, elevated liver enzymes, decreased appetite, and hypertension.

Therefore, developing safe and effective drugs for pulmonary fibrosis holds significant social and medical importance. We have newly discovered that inhibiting the activity of the neurokinin receptor 1 (Tachykinin Neurokinin 1 Receptor, NK-1R) can reduce the production of transforming growth factor-β1 (TGF-β1) in bronchial epithelial cells, suggesting that this target has potential for the development of anti-pulmonary fibrosis drugs.

Vofopitant((2S,3S)-N-{2-methoxy-5-[5-(trifluoromethyl)-1H-tetrazol-1-yl]benzy 1}-2-phenyl-3-piperidinamine), also known as vofopitant, is a potent, selective, and orally active tachykinin NK1 receptor antagonist used as an antiemetic. There is currently no report on the use of Vofopitant for treating pulmonary fibrosis. Additionally, Vofopitant still suffers from low metabolic stability and relatively low bioavailability.

Given the importance of developing structurally novel anti-pulmonary fibrosis drugs, there is an urgent need to develop an anti-pulmonary fibrosis compound with a novel structure, good therapeutic efficacy, and excellent pharmacokinetic properties.

### SUMMARY OF THE INVENTION

The technical problem to be solved by the present invention is to overcome the defects of existing anti-pulmonary fibrosis compounds, such as relatively single structure, poor metabolic stability, and relatively low bioavailability. To this end, the present invention provides a tetrazole compound, a pharmaceutically acceptable salt thereof, and uses thereof, which have one or more of the following advantages: novel structure, good metabolic stability, high bioavailability, and effectively alleviating pathological damage caused by pulmonary fibrosis.

The present invention solves the above technical problems through the following technical solutions.

The present invention provides a compound I, a pharmaceutically acceptable salt thereof, a solvate thereof, or a solvate of a salt thereof,

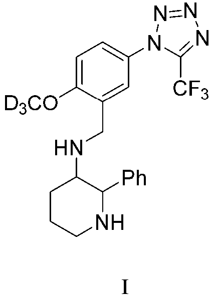

In a certain aspect of the present invention, the compound I is selected from the following structures:

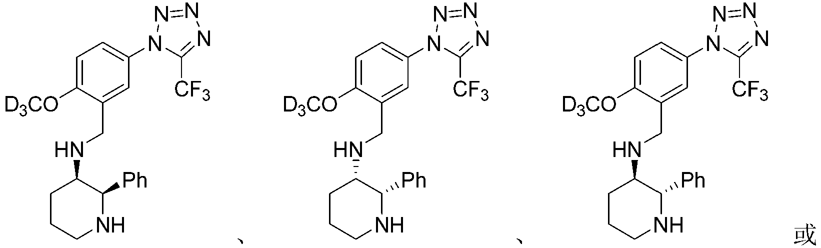

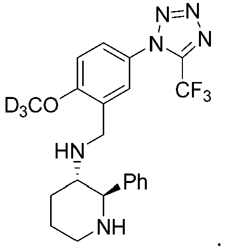

The present invention also provides a pharmaceutical composition, comprising substance D and a pharmaceutical excipient, wherein substance D is the compound I, a pharmaceutically acceptable salt thereof, a solvate thereof, or a solvate of a salt of the compound I.

The present invention also provides a use of substance D in the manufacture of a medicament for treating pulmonary fibrosis, wherein substance D is the compound I, a pharmaceutically acceptable salt thereof, a solvate thereof, or a solvate of a salt of the compound I, or the pharmaceutical composition.

The pulmonary fibrosis is preferably pulmonary interstitial fibrosis or idiopathic pulmonary fibrosis.

The present invention also provides a use of substance D in the manufacture of a medicament for treating a disease associated with NK-1R, wherein substance D is the compound I, a pharmaceutically acceptable salt thereof, a solvate thereof, or a solvate of a salt of the compound I, or the pharmaceutical composition;
the disease associated with NK-1R is preferably nausea and/or vomiting; more preferably chemotherapy-induced nausea and/or vomiting.

The present invention also provides a use of substance D in the manufacture of a medicament for treating nausea and/or vomiting, wherein substance D is the compound I, a pharmaceutically acceptable salt thereof, a solvate thereof, or a solvate of a salt of the compound I, or the pharmaceutical composition;
said nausea and/or vomiting is preferably chemotherapy-induced nausea and/or vomiting.

The present invention also provides a use of substance D in the manufacture of an NK-1R inhibitor, wherein substance D is the compound I, a pharmaceutically acceptable salt thereof, a solvate thereof, or a solvate of a salt of the compound I, or the pharmaceutical composition.

The present invention also provides a compound A, a salt thereof, a solvate thereof, or a solvate of a salt thereof,

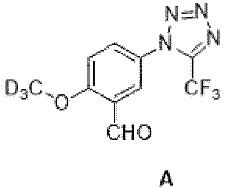

As used herein, "pharmaceutically acceptable salt" refers to a salt that retains the biological activity of the present compound and does not cause toxic side effects. Pharmaceutically acceptable salts include various crystalline forms and amorphous forms of different salts. The pharmaceutically acceptable salts of the tetrazole compounds of the present invention can be formed with inorganic or organic acids. Compared with the free form of the active compound, the pharmaceutically acceptable salt may impart improved pharmacokinetic properties to the active compound. The pharmaceutically acceptable salt may also impart desired pharmacokinetic properties that the active compound did not previously possess, and may even positively affect the pharmacodynamics of the active compound with respect to its therapeutic activity in vivo.

The medicament or pharmaceutical composition of the present disclosure may comprise a pharmaceutically acceptable carrier.

The term "carrier" as used in the present disclosure includes acceptable diluents, excipients, adjuvants, media, solubilizing aids, viscosity modifiers, preservatives, and other known agents used to provide favorable properties in the final medicament or pharmaceutical composition administered to a subject.

Pharmaceutical formulations can be prepared by any method known in the pharmaceutical art. Generally, the active compound can be mixed uniformly and intimately with a liquid carrier, a solid carrier, or both, and then, if necessary, the product can be shaped or packaged into the desired formulation.

The medicament or pharmaceutical composition of the present disclosure may also be a formulation for administration by inhalation. When the medicament or pharmaceutical composition of the present disclosure is for administration by inhalation, it may be in dry powder form, in the form of an aqueous solution or suspension, or in the form of an aerosol.

The medicament or pharmaceutical composition in dry powder form may contain suitable carriers such as lactose or starch, and may be present in different primary packaging systems (e.g., capsules and cartridges or blisters) for use in an inhaler or insufflator. The packaging of the formulation may be adapted for unit dose or multi-dose delivery. In the case of multi-dose delivery, the formulation may be pre-metered or metered at the time of use.

The medicament or pharmaceutical composition of the present disclosure may be in unit dosage form, such as tablets, capsules, or metered aerosol doses, so that a single dose can be administered to a subject.

The medicament or pharmaceutical composition of the present disclosure can be administered by a single route or by a combination of multiple routes, depending on whether local or systemic treatment is required and the area to be treated. When administered in combination in different dosage forms, they can be administered simultaneously, or at closely spaced or widely spaced time intervals, for example, one dosage form in the morning and another in the evening.

The exact amount of the medicament or pharmaceutical composition of the present disclosure required to obtain a therapeutic effect will vary from subject to subject, depending on the subject's species, age, weight, and general condition, the severity of the condition being treated, the particular active agent used, its mode of administration, and the like. The dosage range for administration of the medicament or pharmaceutical composition of the present disclosure is large enough to produce a therapeutic effect. Dosage can be adjusted to avoid or reduce adverse side effects, such as the occurrence of unwanted cross-reactions, allergic reactions, and the like. Dosage may vary depending on the patient's age, condition, sex, and extent of disease, route of administration, or whether other drugs are included in the treatment regimen. In the event of any contraindication, the dosage may be adjusted by the individual physician. Dosage can vary and can be administered one or more doses per day, for one or several days. Guidelines for suitable dosages for a given type of pharmaceutical product can be found in the literature.

In the medicament or pharmaceutical composition of the present disclosure, compounds 1-4 may be the only active ingredients for treating pulmonary fibrosis, or the medicament or pharmaceutical composition of the present disclosure may further contain other active ingredients for treating pulmonary fibrosis. The other active ingredients may be any known drug with activity for treating pulmonary fibrosis.

When the subject also has other diseases, disorders, or conditions, the medicament or pharmaceutical composition of the present disclosure may be administered concurrently with, or at closely spaced or widely spaced time intervals from, drugs used to treat these diseases, disorders, or conditions.

The present invention has been described in detail above, but the above embodiments are merely illustrative in nature and are not intended to limit the present invention. Furthermore, the present text is not to be limited by any theory described in the foregoing prior art, summary of the invention, or the following examples.

Without departing from common general knowledge in the art, the above preferred conditions can be combined arbitrarily to obtain preferred examples of the present invention.

The reagents and raw materials used in the present invention are all commercially available.

The positive progressive effects of the present invention are: the present invention discloses a tetrazole compound and a pharmaceutically acceptable salt thereof, which have one or more of the following advantages: novel structure, good metabolic stability, high bioavailability, and effectively alleviating pathological damage caused by pulmonary fibrosis.

### DETAILED DESCRIPTION OF EMBODIMENTS

The present invention is further illustrated by the following examples, but the present invention is not limited to the scope of the described examples. The experimental methods in the following examples, for which specific conditions are not indicated, are carried out according to conventional methods and conditions, or according to the product specifications.

The structures of the compounds were determined by nuclear magnetic resonance (NMR) or mass spectrometry (MS). NMR was measured using a Bruker ASCEND-400 nuclear magnetic resonance instrument. Solvents were deuterated dimethyl sulfoxide (DMSO-d6), deuterated chloroform (CDCl₃), or deuterated methanol (CD₃OD). Tetramethylsilane (TMS) was used as an internal standard. Chemical shifts are given in units of 10⁻⁶ (ppm).

MS was determined using a Waters SQD (ESI) mass spectrometer (manufacturer: Agilent, model: 6120).

Known starting materials of the present invention can be synthesized by or according to methods known in the art, or can be purchased from Acros Organics, Sigma-Aldrich Chemical Company, Energy Chemical, PharmaBlock Sciences, and other companies.

Unless otherwise specified in the following examples, reactions were carried out under an argon or nitrogen atmosphere.

An argon or nitrogen atmosphere means that the reaction flask is connected to an argon or nitrogen balloon, for example, a 1 L argon or nitrogen balloon.

A hydrogen atmosphere means that the reaction flask is connected to an argon balloon, for example, a 1 L argon balloon.

Hydrogenation reactions are typically evacuated, filled with hydrogen, and the operation is repeated three times.

### Example 1: Synthesis of Intermediate A

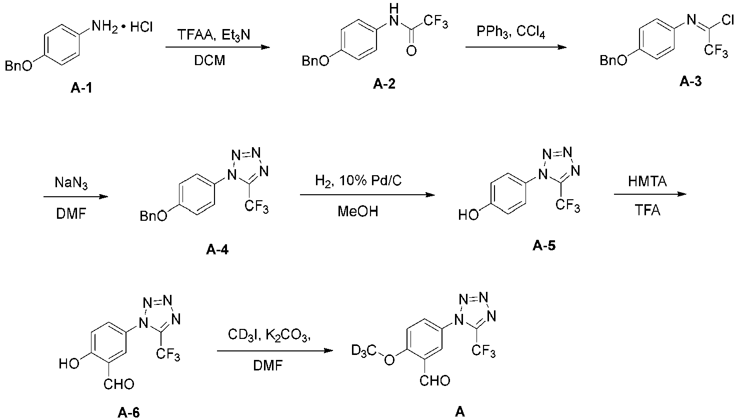

The synthesis schemes for intermediates A-2 to A-6 refer to patent WO0018403A1.

### Synthesis of Intermediate A:

Intermediate A-6 (2 mmol) was dissolved in DMF (5 mL). K₂CO₃ (3 mmol) and CD₃I (3 mmol) were added to the solution. The reaction was stirred at room temperature for 2 hours. TLC showed complete conversion of the starting material. The reaction was quenched by adding water (20 mL) in an ice bath. The mixture was extracted three times with ethyl acetate (15 mL). The combined organic phases were washed sequentially with water (50 mL) and saturated brine (50 mL), dried over Na₂SO₄, and concentrated to give a crude product. The crude product was purified by silica gel column chromatography (PE/EA=5:1) to obtain a white solid product.

¹H NMR (400 MHz, d6-DMSO) δ 7.23 (d, 1H), 7.62 (dd, 1H), 7.73 (d, 1H), 9.96 (s, 1H). LC-MS: M/Z (ESI): 276.1 (M+1)

### Example 2: Synthesis of Compound 1 ((2R,3R)-2-phenyl-3-[({2-[(trideuteriomethyl)oxy]-5-[5-(trifluoromethyl)-1,2,3, 4-tetrazol-1-yl]phenyl}methyl)amino]piperidine)

The synthetic route for Compound 1 is as follows:

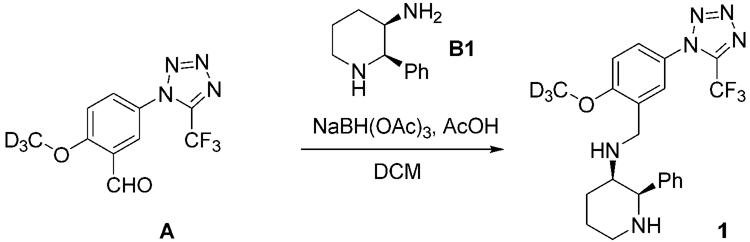

### Preparation of NaBH(OAc)₃ solution:

Under N₂ protection, NaBH₄ (5 mmol) was added to THF (10 mL) and stirred at 0°C for 15 minutes. At 0°C, AcOH (20 mmol) was slowly added dropwise to the reaction solution (exothermic during addition, with a large amount of gas bubbles generated; control the addition rate). After the addition was complete, the mixture was stirred at 0°C for 15 minutes until no more gas was generated. AcOH (20 mmol) was then added, and the reaction mixture was allowed to warm to room temperature and stirred for 30 minutes to obtain the desired NaBH(OAc)₃ solution, which was used directly in the subsequent reaction.

### Preparation of Compound 1:

Under N₂ protection, intermediate A (1 mmol) and intermediate B1 (1.05 mmol) were dissolved in dichloromethane (25 mL). AcOH (10 mmol) was added to the reaction solution, and the mixture was stirred at room temperature for 30 minutes. The reaction solution was transferred to an ice bath at 0°C, and the NaBH(OAc)₃ solution prepared above was added dropwise to the reaction solution. After the addition was complete, the mixture was allowed to warm to room temperature and stirred for 18 hours. TLC confirmed complete conversion of the starting material. The mixture was transferred to an ice bath, and 2N Na₂CO₃ solution (50 mL) was slowly added to quench the reaction, with a large amount of gas generated. After stirring until no more gas was generated, the mixture was extracted three times with dichloromethane (25 mL). The combined organic phases were washed sequentially with water (50 mL) and saturated brine (50 mL), dried over Na₂SO₄, and concentrated to give a crude product. The crude product was purified by silica gel column chromatography (DCM/MeOH=20:1) to obtain a colorless to pale yellow transparent oil product. LC-MS: M/Z (ESI): 436.1 (M+1)

### Example 3: Synthesis of Compound 2 ((2S,3S)-2-phenyl-3-[({2-[(trideuteriomethyl)oxy]-5-[5-(trifluoromethyl)-1,2,3,4 -tetrazol-1-yl]phenyl} methyl)amino]piperidine)

The synthetic route for Compound 2 is as follows:

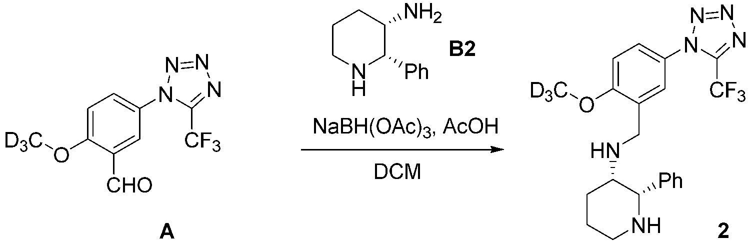

According to the synthetic method in Example 2, Compound 2 was synthesized from Intermediate A and Intermediate B2. LC-MS: M/Z (ESI): 436.1 (M+1)

### Example 4: Synthesis of Compound 3 ((2S,3R)-2-phenyl-3-[({2-[(trideuteriomethyl)oxy]-5-[5-(trifluoromethyl)-1,2,3, 4-tetrazol-1-yl]phenyl}methyl)amino]piperidine)

The synthetic route for Compound 3 is as follows:

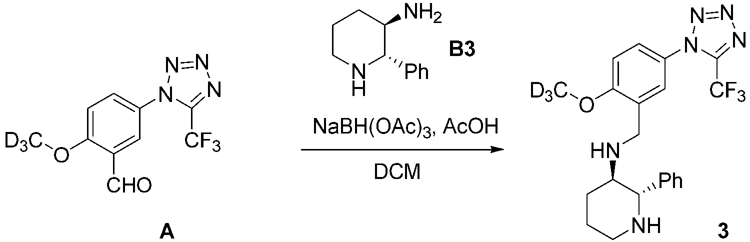

According to the synthetic method in Example 2, Compound 3 was synthesized from Intermediate A and Intermediate B3. LC-MS: M/Z (ESI): 436.1 (M+1)

### Example 5: Synthesis of Compound 4 ((2R,3S)-2-phenyl-3-[({2-[(trideuteriomethyl)oxy]-5-[5-(trifluoromethyl)-1,2,3, 4-tetrazol-1-yl]phenyl}methyl)amino]piperidine)

The synthetic route for Compound 4 is as follows:

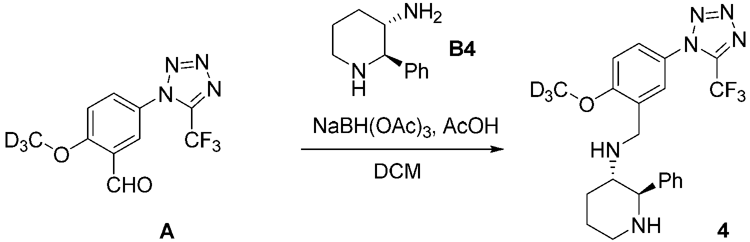

According to the synthetic method in Example 2, Compound 4 was synthesized from Intermediate A and Intermediate B4. LC-MS: M/Z (ESI): 436.1 (M+1)

### Example 6: Pharmacodynamic Experiments

The oral medicaments in this example comprise an active pharmaceutical ingredient and a solvent.

The active pharmaceutical ingredient is Compound 1 to Compound 4, at a dosage of 30 mg/kg. The solvent is water.

Pulmonary fibrosis model establishment: Male C57BL6J mice (Vital River, C57BL/6JNifdc), aged 6-8 weeks, were adaptively fed for one week and divided into a model group (6 mice), a Compound 1 treatment group (6 mice), a Compound 2 treatment group (6 mice), a Compound 3 treatment group (6 mice), a Compound 4 treatment group (6 mice), a Vofopitant treatment group (6 mice), a Nintedanib (Nintedanib is a commonly used drug in the prior art for treating IPF) treatment group (6 mice), and a control group (6 mice).

Preparation of bleomycin solution: Bleomycin dry powder was prepared to the desired concentration using sterile PBS.

Non-surgical method for intratracheal instillation of bleomycin into mouse lungs: Mice were anesthetized with isoflurane and suspended on an operating board at a 70° angle. Bleomycin was instilled intratracheally using a 200 µL pipette.

On the first day, the model group and each treatment group received intratracheal instillation of bleomycin solution (1 mg/kg/50 µL). The control group received an equal volume of normal saline. Treatment began 7 days after model establishment and continued for 14 days, totaling 21 days.

### Preparation and administration of Compound 1 to Compound 4 drug solutions:

Compounds 1 to 4 were each dissolved in water to prepare 30 mg/mL stock solutions. For working solutions, the stock solutions were diluted 5-fold with autoclaved water to the required volume. Each mouse received Compound 1 to Compound 4 at a dose of 30 mg/kg, administered by gavage.

### Preparation and administration of Vofopitant drug solution:

Vofopitant was dissolved in water to prepare a 30 mg/mL stock solution. For the working solution, the stock solution was diluted 5-fold with autoclaved water to the required volume. Each mouse received Vofopitant at a dose of 30 mg/kg, administered by gavage.

Preparation, dosage, and administration of Nintedanib drug solution: Nintedanib was dissolved in DMSO to prepare a stock solution (40 mg/mL). For the working solution, the stock solution was diluted 7-fold with 20% SBE-β-CD physiological saline solution to the required volume and mixed well. Each mouse received Nintedanib at a dose of 30 mg/kg, administered by gavage.

Evaluation of the effects of Compound 1 to Compound 4 treatment, Nintedanib treatment, and Vofopitant treatment on survival rate and lung injury in pulmonary fibrosis mice.
(1) Treatments with Compounds 1 to 4 significantly increased the survival rate of bleomycin-induced pulmonary fibrosis mice.

As shown in Table 1, compared with the control group, all mice in the model group died. Compared with the model group, the treatment groups with Compounds 1 to 4 significantly increased the survival rate of pulmonary fibrosis mice. The survival rates of mice in the Vofopitant treatment group and the Nintedanib treatment group were also improved. As shown in Table 1, all mice in the model group died, while the survival rates of mice in the Compound 1 to Compound 4 groups, Vofopitant group, and Nintedanib group were all increased. Furthermore, the effects of Compounds 1 to 4 were 1.2 times that of the Vofopitant group and 1.6 times that of the Nintedanib group.

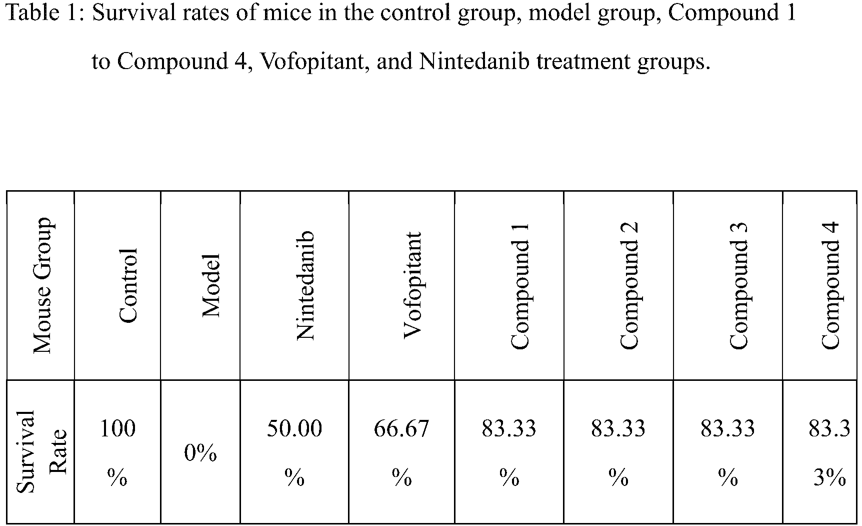

(2) Treatments with Compounds 1 to 4 alleviated the degree of pulmonary fibrosis.

MASSON staining: Mouse lung tissue samples were fixed in 4% paraformaldehyde for 24 hours, dehydrated through a graded alcohol series, embedded in paraffin, cut into 5 µm sections, and subjected to MASSON staining. After clearing with xylene, the sections were mounted with neutral balsam. Pathological changes in the lungs were observed and photographed under an optical microscope. Pathological images were scored using the modified Ashcroft scale system, as shown in Table 2 below.

The scoring criteria for the modified Ashcroft scale are as follows:

| Fibrosis Grade | Alveolar Septa | Lung Structure |
|---|---|---|
| 0 | No fibrosis | Normal lung |
| 1 | Isolated mild fibrotic changes (septa ≤ 3 times normal thickness) | Alveoli partially enlarged or thinned, but no fibrotic masses |
| 2 | Obvious fibrotic changes (>3 times normal thickness) with nodular structures but not interconnected | Alveoli partially enlarged or thinned, but no fibrotic masses |
| 3 | Continuous fibrotic walls throughout the microscopic field (>3 times normal thickness) | Alveoli partially enlarged or thinned, but no fibrotic masses |
| 4 | Variable | Single fibrotic mass (≤10% of the field) |
| 5 | Variable | Confluent fibrotic masses (10% < field ≤ 50%), severe disruption of lung structure but some preservation |
| 6 | Variable, mostly absent | Large continuous fibrotic masses (>50% of the field), lung structure mostly not preserved |
| 7 | Absent | Almost completely replaced by fibrotic masses, still with up to 5 bubble structures |
| 8 | Absent | Entire field completely occupied by fibrotic masses |

As shown by the scoring results in Table 2, compared with the control group, the degree of pulmonary fibrosis injury in the model group mice was exacerbated. The Compound 1 to Compound 4 treatment groups, the Vofopitant treatment group, and the Nintedanib treatment group all ameliorated the degree of pulmonary fibrosis injury in mice. Moreover, the improvement in pulmonary fibrosis injury in mice achieved by Compounds 1 to 4 was superior to that of the Nintedanib group and the Vofopitant group.

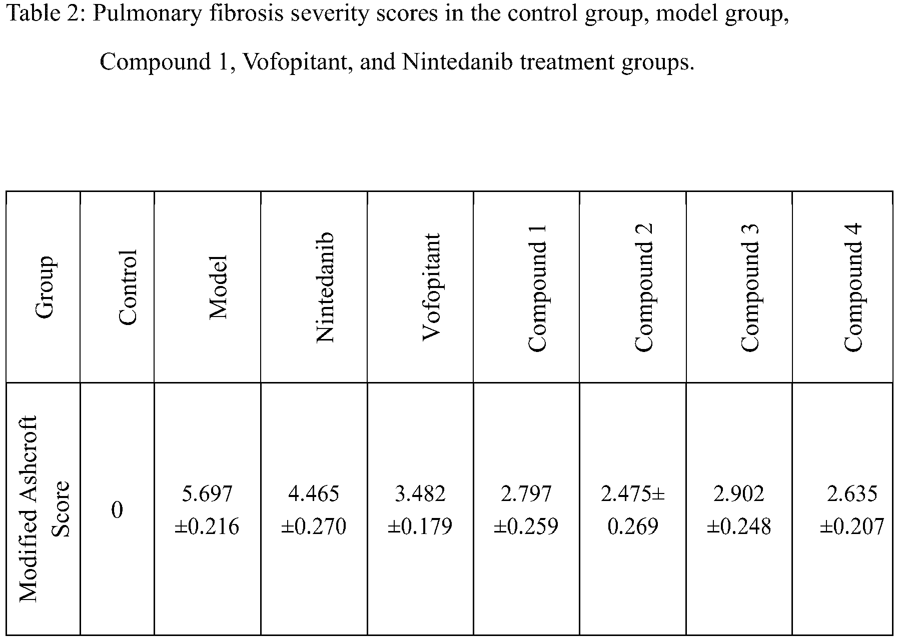

### Example 7: Rat Pharmacokinetic Studies

For the rat pharmacokinetic study, male SD rats (Shanghai Jihui) weighing 200-250 g each were fasted overnight. Six rats were administered Compounds 1-4 of the present invention and Vofopitant separately by oral gavage at a dose of 10 mg/kg, with water as the vehicle. Blood samples were collected before administration and at 15 min, 30 min, 1 h, 2 h, 4 h, 6 h, 8 h, 10 h, and 24 h after administration. Blood samples of 0.2 mL were collected into labeled tubes containing EDTA-2K anticoagulant. The tubes were gently inverted to mix the anticoagulant (EDTA-2K) thoroughly with the blood, immediately placed in wet ice, and centrifuged to separate plasma within 1 hour after blood collection. Centrifugation conditions were 4°C, 6800 × g, for 6 minutes. The separated plasma was transferred to labeled Eppendorf tubes and stored in an ultra-low temperature freezer as soon as possible until sample analysis. Major pharmacokinetic parameters were analyzed using WinNonlin 7.0 software with a non-compartmental model. The results are shown in Table 3.

**Table 3: Rat pharmacokinetic study results.**

| Test Compound | Rat Pharmacokinetic Parameters (Oral Gavage Administration) | | | | |
|---|---|---|---|---|---|
| | Cₘₐₓ (ng/mL) | Tₘₐₓ (hr) | AUC₀₋₂₄ₕ (h*ng/mL) | T_{1/2} (hr) | F (%) |
| Vofopitant | 929 | 0.5 | 3367 | 1.67 | 56.5 |
| Compound 1 | 1164 | 0.25 | 3708 | 1.60 | 74.3 |
| Compound 2 | 1210 | 0.25 | 3867 | 1.70 | 78.7 |
| Compound 3 | 972 | 0.25 | 3216 | 1.38 | 65.5 |
| Compound 4 | 1310 | 0.25 | 3873 | 1.73 | 78.8 |

Herein, Cₘₐₓ represents peak concentration, Tₘₐₓ represents time to peak concentration, AUC represents area under the plasma concentration-time curve, T_{1/2} represents elimination half-life, and F represents bioavailability.

The experimental results demonstrate that Compounds 1 to 4 of the present invention exhibit excellent plasma exposure, excellent bioavailability, and favorable pharmacokinetic properties.

Although specific embodiments of the present invention have been described above, those skilled in the art should understand that these are merely illustrative and that various changes or modifications can be made to these embodiments without departing from the principles and essence of the present invention. Therefore, the scope of protection of the present invention is defined by the appended claims.

## Claims

1. A compound I, a pharmaceutically acceptable salt thereof, a solvate thereof, or a solvate of a salt thereof,

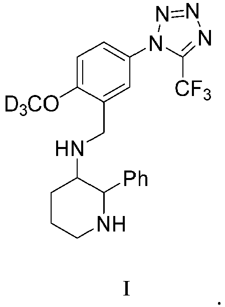

2. The compound I, a pharmaceutically acceptable salt thereof, a solvate thereof, or a solvate of a salt thereof according to claim 1, wherein the compound I is selected from the following structures: 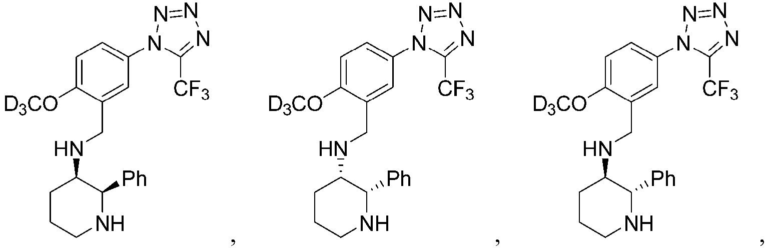 or

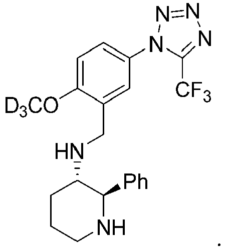

3. A pharmaceutical composition, comprising substance D and one or more pharmaceutical excipients, wherein substance D is the compound I, a pharmaceutically acceptable salt thereof, a solvate thereof, or a solvate of a salt thereof according to any one of claims 1-2.

4. A use of substance D in the manufacture of a medicament for treating pulmonary fibrosis, wherein substance D is the compound I, a pharmaceutically acceptable salt thereof, a solvate thereof, or a solvate of a salt of the compound I according to any one of claims 1-2, or the pharmaceutical composition according to claim 3.

5. The use according to claim 4, wherein the pulmonary fibrosis is pulmonary interstitial fibrosis or idiopathic pulmonary fibrosis.

6. A use of substance D in the manufacture of a medicament for treating a disease associated with NK-1R, wherein substance D is the compound I, a pharmaceutically acceptable salt thereof, a solvate thereof, or a solvate of a salt of the compound I according to any one of claims 1-2, or the pharmaceutical composition according to claim 3.

7. The use according to claim 6, wherein the disease associated with NK-1R is nausea and/or vomiting; preferably chemotherapy-induced nausea and/or vomiting.

8. A use of substance D in the manufacture of a medicament for treating nausea and/or vomiting, wherein substance D is the compound I, a pharmaceutically acceptable salt thereof, a solvate thereof, or a solvate of a salt of the compound I according to any one of claims 1-2, or the pharmaceutical composition according to claim 3;
said nausea and/or vomiting is preferably chemotherapy-induced nausea and/or vomiting.

9. A use of substance D in the manufacture of an NK-1R inhibitor, wherein substance D is the compound I, a pharmaceutically acceptable salt thereof, a solvate thereof, or a solvate of a salt of the compound I according to any one of claims 1-2, or the pharmaceutical composition according to claim 3.

10. A compound A, a salt thereof, a solvate thereof, or a solvate of a salt thereof,
